# EUROPEAN PATENT APPLICATION

(11) **EP 4 201 378 A1**
(43) Date of publication of application: **28.06.2023**
(21) Application number: 21857184.2
(22) Date of filing: 06.04.2021
(51) Int. Cl.: A61F 2/24

(54) **VALVE CLAMPING DEVICE HAVING LOCKING MECHANISM, AND VALVE REPAIRING SYSTEM**

(30) Priority: 21.08.2020 CN 202010855704; 21.08.2020 CN 202021777931 U
(71) Applicant: Hangzhou Valgen Medtech Co., Ltd., Hangzhou, Zhejiang 310051 (CN)
(72) Inventor: ZHANG, Tingchao, Hangzhou, Zhejiang 310051 (CN); ZHENG, Xianzhang, Hangzhou, Zhejiang 310051 (CN); ZHANG, Weiwei, Hangzhou, Zhejiang 310051 (CN)
(74) Representative: HGF
(86) International application number: PCT/CN2021/085735
(87) International publication number: WO 2022/037083

(57) **Abstract**

The present invention discloses a valve clamping device (100) with locking mechanism (80), and a valve repairing system. The valve clamping device (100) includes a fixing base (20), at least one pair of clamping arms (40), an actuator assembly (70) and a locking mechanism (80), the at least one pair of clamping arms (40) being connected to the fixing base (20) and being able to be open and closed relative to the fixing base (20). The actuator assembly (70) includes an actuator shaft (72) movably inserted into the fixing base (20), the actuator shaft (72) moves axially to actuate the clamping arms (40) to be open and closed relative to the fixing base (20), and a positioning portion (720) is provided on the outer peripheral surface of the actuator shaft (72). The locking mechanism (80) includes a locking member (82) and a pushing member (84), a locking hole (820) is provided axially in the locking member (82), the actuator shaft (72) passes through the locking hole (820), and the pushing member (84) abuts against the locking member (82) and is provided obliquely in the fixing base (20), so that an edge (281) of the locking hole (820) is engaged with the positioning portion (720). The locking mechanism (80) can increase a friction force and a mechanical engagement force between the actuator shaft (72) and the locking member (82), and prevent the edge (281) of the locking hole of the locking member (82) from being locally stressed and wearing, improving the anti-fatigue performance of the valve clamping device (100), improving the locking stability, and preventing locking failure.

## Description

### TECHNICAL FIELD

The present application relates to the field of interventional medical devices, and in particular, to a valve clamping device with a locking mechanism, and a valve repair system.

### BACKGROUND

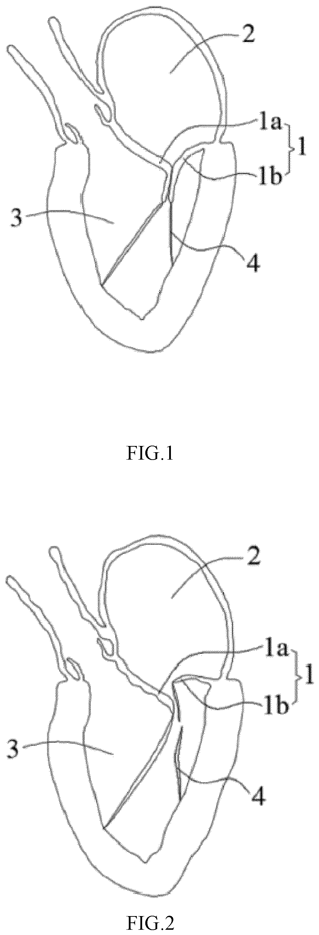

Please refer to FIG.1, the mitral valve 1 is a one-way valve located between the left atrium 2 and the left ventricle 3 of the heart. A normal healthy mitral valve 1 can control the blood flow from the left atrium 2 to the left ventricle 3, while preventing blood from flowing back into left atrium 2. The mitral valve 1 includes a pair of leaflets, referred to as an anterior leaflet 1a and a posterior leaflet 1b. The anterior leaflet 1a and the posterior leaflet 1b are connected to the papillary muscle of the left ventricle 3 through the chordae tendineae 4. Under normal circumstances, when the heart contracts, the edges of the anterior leaflet 1a and the posterior leaflet 1b are completely aligned, preventing blood from flowing back into the left atrium 2. Please refer to FIG.2, when the leaflets of mitral valve 1 or their related structures change qualitatively or functionally, such as partial rupture of chordae tendineae 4, the anterior leaflet 1a and posterior leaflet 1b of mitral valve 1 are misaligned Therefore, when the heart contracts, the mitral valve 1 cannot be completely closed, resulting in the backflow of blood from the left ventricle 3 to the left atrium 2, thereby causing a series of pathophysiological changes, called "mitral regurgitation".

Surgical procedures such as edge-to-edge repairingare usually used to treat mitral regurgitation. However, this type of surgery has disadvantages such as complex surgical procedure, high surgical cost, severe trauma, high risk of complications, long hospital stay, and painful recovery process for patients. Currently, there is a minimally invasive medical device, which is based on the principle of edge-to-edge repair of the mitral valve, and delivers a valve clamping device to the mitral valve through an interventional catheter, and then simultaneously grasps the anterior leaflet 1a and posterior leaflet 1b of the mitral valve through the relative open and close of the valve clamp, thereby pulling the leaflets toward each other, achieve the purpose of reducing the leaflet gap and treating mitral regurgitation.

Currently, there is a valve clamping device with a locking mechanism. The locking mechanism includes a wedge-shaped element and a metal frame that overlaps with the wedge-shaped element. A hole is formed in the wedge-shaped element, and an actuator shaft of the valve clamping device extends through the hole. When the metal frame is pulled towards a proximal end, one end of the wedge-shaped element is pulled and the other end is stationary, thereby reducing the friction between the actuator shaft and the through hole, so that the actuator shaft can move in the through hole. However, since the surface of the actuator shaft and the inner surface of the through hole of the wedge-shaped element are both smooth, the friction force between the two is small, so the locking force is small, and the locking mechanism may wear, slip or fail, resulting in the valve clamping device is opened ahead of time during the delivery process, or accidentally opened during the process of clamping the valve leaflets, or the valve clamping device falls off after implantation, resulting in failure of the operation.

### SUMMARY

In view of this, the present application provides a valve clamping device with a locking mechanism, which can improve the locking force of the locking mechanism, prevent locking failure, and improve the safety and fatigue resistance of the valve clamping device.

In order to solve the above-mentioned technical problems, the present application provides a valve clamping device with a locking mechanism, including a fixing base, at least one pair of clamping arms, an actuator assembly, and a locking mechanism. The at least one pair of clamping arms is connected to the fixing base, and can be open and close relative to the fixing base. The actuator assembly includes an actuator shaft movably inserted in the fixing base, and the actuator shaft moves axially to actuate the clamping arm open or close relative to the fixing base. The outer peripheral surface of the actuator shaft is provided with a positioning portion. The locking mechanism includes a locking member and a pushing member, the locking member is provided with a locking hole along the axial direction, and the actuator shaft is inserted through the locking hole. The pushing member abuts the locking member and is obliquely disposed in the fixing base, so that the edge of the locking hole is engaged to the positioning portion.

The present application also provides a valve repair system, including a valve clamping device and a delivery device detachably connected to the valve clamping device, the delivery device includes an operating line, the distal end of the operating line is detachably connected to the unlocking member.

The outer peripheral surface of the actuator shaft of the valve clamping device provided by the present application is provided with a positioning portion, the actuator shaft is inserted into the locking member, and the pushing member abutting the locking member is obliquely arranged in the fixing base, so that the edge of the locking hole is engaged to the positioning portion. In this way, a frictional force and mechanical engagement can be improved between the actuator shaft and locking member, this not only prevent the edge of the locking hole of the locking member from being partially stressed and wear out, but also the edge of the actuator shaft and the locking hole have a certain amount of engagement, therefore, the two will be more tightly engaged under forces, and the actuator shaft and the edge of the locking hole are not matched only by frictional force alone, which improves the locking force and stability of the locking mechanism and prevents the valve clamping device from locking failure.

### BRIEF DESCRIPTION OF THE ACCOMPANYING DRAWINGS

In order to illustrate the technical solutions of the embodiments of the present disclosure more clearly, the following briefly introduces the accompanying drawings that need to be used in the embodiments. As far as technical personnel are concerned, other drawings can also be obtained based on these drawings without any creative effort.
FIG. 1 is a schematic view of the mitral valve in a normal state.
FIG.2 is a schematic view of the mitral valve with lesions.
FIG.3 is a schematic three-dimensional structural view of a valve clamping device with a locking mechanism provided by the first embodiment of the present application.
FIG.4 is a side view of the valve clamping device with locking mechanism of FIG.3.
FIG.5 is a schematic three-dimensional structural view of the fixing base, part of the actuator assembly and the locking mechanism of the valve clamping device in FIG.3.
FIG.6 is a side view of the fixing base, part of the actuator assembly and the locking mechanism of the valve clamping device of FIG.5.
FIG.7 is an enlarged view of part VII in FIG.6.
FIG.8 is a cross-sectional view of FIG.5 with the pushing member and the unlocking member being omitted.
FIG.9 is an enlarged view of part IX in FIG.8.
FIG. 10 is a schematic three-dimensional structural view of the actuator shaft and the connection base of the valve clamping device in FIG.3.
FIG.11 is a schematic three-dimensional structural view of the locking member in FIG. 5.
FIG. 12 is a schematic three-dimensional structural view of the pushing member in FIG. 5.
FIG. 13 is a schematic three-dimensional structural view of another embodiment of the pushing member in FIG. 12.
FIG. 14 is a perspective structural schematic view of the locking mechanism in the unlocking state of the valve clamping device in FIG.5.
FIG. 15 is a side view of the locking mechanism in the unlocked state of the valve clamping device of FIG. 14.
FIG. 16 is an enlarged view of part XVI in FIG. 15.
FIG. 17 is a schematic three-dimensional structural view of the unlocking member in FIG. 5.
FIG. 18 is a side view of the unlocking member of FIG. 17.
FIG. 19 is a schematic three-dimensional structural view of the fixing base, part of the actuator assembly, the locking mechanism and the clamping arm in FIG.5.
FIG.20 is a side view of FIG. 19.
FIG.21 is a schematic view of the valve clamping device with locking mechanism in one the use states in FIG.3.
FIG.22 is a schematic view of the valve clamping device with a locking mechanism in the closed state in FIG.3.
FIG.23 is a schematic three-dimensional structural view of a valve clamping device with a locking mechanism provided by the second embodiment of the present application.
FIG.24 is a side view of the valve clamping device with locking mechanism of FIG.23.
FIG.25 is a schematic structural view of the fixing base, part of the actuator assembly and the locking mechanism of the valve clamping device with locking mechanism in FIG.23.
FIG.26 is a partial cross-sectional view of the valve clamping device of FIG.25.
FIG.27 is an enlarged view of part XXVII in FIG.26.
FIG.28 is a schematic structural view of a valve clamping device with a locking mechanism provided by the third embodiment of the present application.
FIG.29 is a schematic structural view of a valve clamping device with a locking mechanism provided by the fourth embodiment of the present application.
FIG.30 is a statistical chart of the results of the performance test of the valve clamping device of the present application.

### DETAILED DESCRIPTION OF ILLUSTRATED EMBODIMENTS

The technical solutions in the embodiments of the present application will be clearly and completely described below with reference to the drawings in the embodiments of the present application. Obviously, the described embodiments are only a part of the embodiments of the present application, but not all of the embodiments. Based on the embodiments in the present application, all other embodiments obtained by those of ordinary skill in the art without creative efforts fall within the protection scope of the present application.

In the description of the present application, it should be noted that the orientation or positional relationship indicated by the terms "upper", "lower", "inner", "outer", etc. is based on the orientation or positional relationship shown in the accompanying drawings, only for the purpose of It is for the convenience of describing the present application and simplifying the description, rather than indicating or implying that the referred device or element must have a particular orientation, be constructed and operate in a particular orientation, and therefore should not be construed as a limitation of the present application. Furthermore, the terms "first," "second," etc. are used for descriptive purposes only and should not be construed to indicate or imply relative importance.

In order to describe the structure of the valve clamping device with the locking mechanism and the valve repair system more clearly, the defined terms "proximal end", "distal end" and "axial" described in this application are commonly used terms in the field of interventional medicine. Specifically, the "distal end" refers to the end that is far away from the operator during the surgical operation; the "proximal end" refers to the end that is close to the operator during the surgical operation; the proximal end in this application is relative to the distal distance from the operator ( The distance from the surgeon) is relatively short, and after the device is assembled, each component in the device includes a proximal end and a distal end, wherein the proximal end of each component is closer to the operator than the distal end. "Axial" refers to the direction of the central axis of the device, and the radial direction is the direction perpendicular to the central axis. Unless otherwise defined, all technical and scientific terms used in this application have the same meaning as commonly understood by one of ordinary skill in the technical field to which this application belongs. The conventional terms used in the specification of the present application are only for the purpose of describing specific embodiments, and should not be construed as limitations of the present application.

It should be noted that when an element is referred to as being "fixed to" or "disposed on" another element, the element can be directly connected to the other element or indirectly connected to the other element through one or more connecting elements on a component. When an element is referred to as being "connected to" another element, it can be directly connected to the other element or connected to the other element through one or more connecting elements.

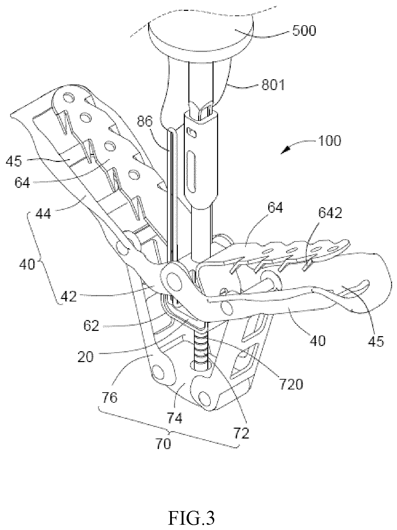

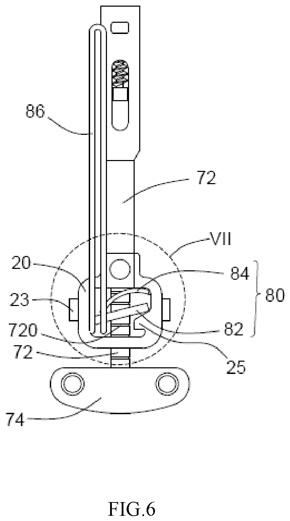

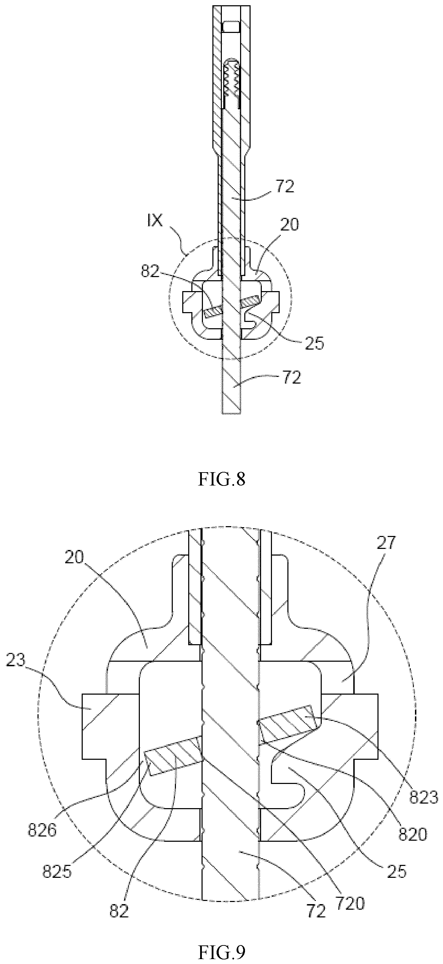

Referring to FIGS. 3-9 together, the first embodiment of the present application provides a valve repair system, which includes a valve clamping device 100 with a locking mechanism, and a delivery device 500 detachably connected to the valve clamping device 100. The valve clamping device 100 includes a fixing base 20, at least a pair of clamping arms 40 hinged with the fixing base 20, a tissue gripper 60 disposed between the fixing base 20 and the clamping arms 40, an actuator assembly 70 used to actuate the clamping arms 40 to be open and close relative to the fixing base 20, and a locking mechanism 80 provided on the fixing base 20. The tissue gripper 60 includes at least a pair of gripping arms 64, each gripping arm 64 is closed to the clamping arm 40 after being released, and cooperates with the clamping arm 40 on the corresponding side to grip the leaflet tissue there between.

Specifically, the actuator assembly 70 includes a actuator shaft 72 movably inserted into the fixing base 20, the actuator shaft 72 moves in the axial direction to actuate the clamping arm 40 to open and close relative to the fixing base 20, and the outer peripheral surface of the actuator shaft 72 is provided with a positioning portion 720. The locking mechanism 80 includes a locking member 82 and a pushing member 84. The locking member 82 defines a locking hole 820 in the axial direction, the actuator shaft 72 is inserted into the locking hole 820, and the pushing member 84 abuts the locking member 82 and is obliquely disposed in the fixing base 20. The edge 281 of the locking hole 820 is engaged with the positioning portion 720, so that the actuator shaft 72 is relatively fixed to the fixing base 20, thereby restricting the relative opening and closing between the clamping arm 40 and the fixing base 20.

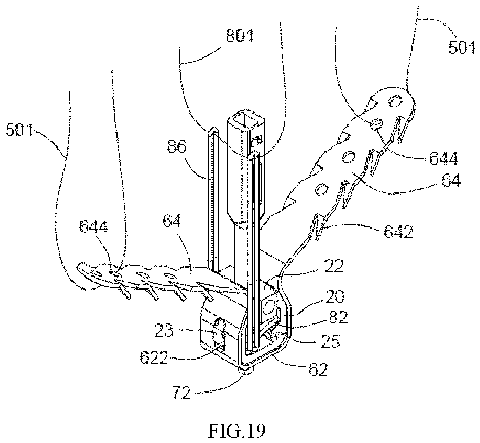

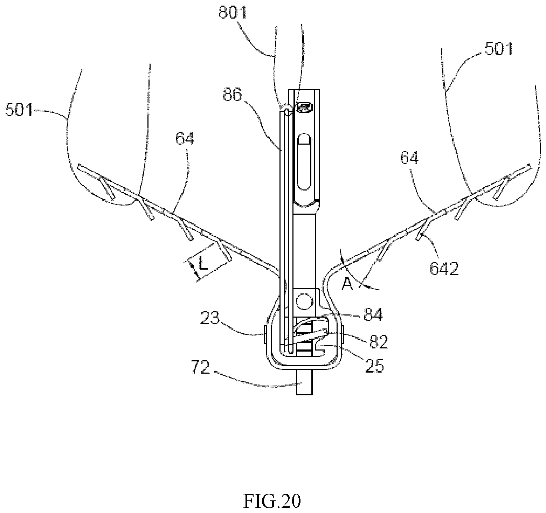

The delivery device 500 includes a tube assembly, an operating line 501 and a control line 801 inserted in the tube assembly, the operating line 501 is connected to the gripping arm 64, the control line 801 is connected to the locking mechanism 80, and the operating line 501 and the control line 801 extend respectively outside the patient's body. In use, the proximal end of the valve clamping device 100 is releasably connected to the distal end of the tube assembly, and the two clamping arms 64 of the valve clamping device 100 are pulled up by the operating line 501 (shown in FIGS. 19 and 20 ) to be closed to the central shaft. At this time, the pushing member 84 abuts the locking member 82 and is obliquely disposed in the fixing base 20, so that the edge 281 of the locking hole 820 is engaged with the positioning portion 720, and restricts the relative movement between the actuator shaft 72 and the fixing base 20 to prevent the clamping arm 40 and the fixing base 20from movement. Then the locking member 82 is pulled from a distance by the control line 801, so that the edge 281 of the locking hole 820 and the actuator shaft 72 can be moved relative to each other. At this time, the locking member 82 exerts pressure on the pushing member 84 to make the pushing member 84 bend and deform, and then operate the actuator shaft 72 to make the clamping arm 40 retract to the outer surface of the fixing base 20, and then release the locking member 82, the pushing member 84 is restored to the initial state and abutting the locking member 82, and the locking member 84is restored to be obliquely disposed in the fixing base 20, the edge 281 of the locking hole 820 is latched to the positioning portion 720, thus the clamping arm 40 and the fixing base 20 are locked. The valve clamping device 100 then can be pushed to the mitral valve of the patient, and after being adjusted to an appropriate position, the locking of the actuator shaft 72 by the locking mechanism 80 is released again, and the actuator shaft 72 is operated to make the clamping arm 40 open relative to the fixing base 20; then the pulling force of the operating line501 on the two clamping arms 64 is released, and the clamping arms 64 spring back and move toward the clamping arms 40 to press the valve leaflets toward the clamping arms 40. When the anterior leaflet and the posterior leaflet of the mitral value is clamped separately by the clamping arm 40 and the corresponding gripping arm 64, the latching of the locking member 82 to the actuator shaft 72 is released, and the actuator shaft 72 is operated so that the clamping arm 40 is closed relative to the fixing base 20 to draw the anterior and posterior leaves toward each other, then the connection between the tube assembly and the valve clamping device 100 is disengaged, the delivery device 500 is withdrawn, and the valve clamping device 100 is left in the patient as an implant in order to keep the coapted positions of the valve leaflets together, achieving "edge-to-edge repair" of the mitral valve to reduce mitral regurgitation in patients.

It should be noted that, the valve clamping device 100 and the delivery device 500 can be delivered into the patient's body by using an available guiding device such as an adjustable sheath, a preformed sheath, and the like.

In the present application, a positioning portion 720 is provided on the outer peripheral surface of the actuator shaft 72 of the valve clamping device 100, the actuator shaft 72 is inserted into the locking hole 820 of the locking member 82, and the pushing member 84 abuts against the locking member 82 and is disposed obliquely in the fixing base 20, thus the edge 281 of the locking hole 820 is stuck and engaged to the positioning portion 720 to increase the friction and mechanical engagement force between the actuator shaft 72 and the locking member 82, which not only prevents the edge of the locking hole 820 of the locking member 82 from being partially affected and abrasion, but also due to the actuator shaft 72 and the edge of the locking hole 820 have a certain amount of engagement, the two will be more tightly engaged under forces. The actuator shaft 72 and the edge of the locking hole 820 do not rely solely on friction, which improves the locking force and stability of the locking mechanism 80 and prevents the valve clamping device 100 from locking failure.

Preferably, the inner diameter of the locking hole 820 of the locking member 82 is appropriately increased. After the actuator shaft 72 is inserted into the locking hole 820, the gap between the outer peripheral surface of the actuator shaft 72 and the inner surface of the locking hole 820 will increase. Therefore, the outer peripheral surface of the actuator shaft 72 and the inner surface of the locking hole 820 can be prevented from interfering with each other due to the overall displacement of the locking member 82 during the unlocking process of the locking member 82.

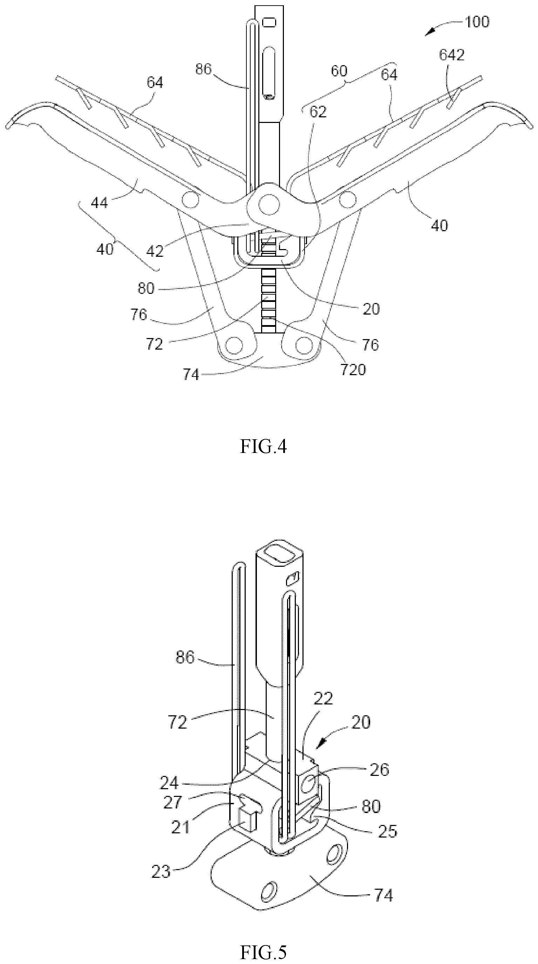

As shown in FIG.3 and FIG.4, in this embodiment, the valve clamping device 100 includes a pair of clamping arms 40 disposed opposite to each other, and each clamping arm 40 can be opened and closed relative to the fixing base 20. Each clamping arm 40 includes a connecting frame 42 and a clamping frame 44 connected to one end of the connecting frame42 away from the fixing base 20. The ends of the connecting frames 42 of the two clamping arms 40 away from the clamping frame 44 are stacked on each other and then hinged to the fixing base 20. A leaflet accommodating space is formed between the gripping arm 64 and the clamping arm 40. Specifically, the surface of each clamping arm 40 facing the gripping arm 64 is recessed inward to form a receiving groove 45, so that when the valve clamping device 100 is in the delivery state, the gripping arm 64 is at least partially accommodated in the receiving groove 45 of the clamping arm 40, so as to reduce the outer diameter and volume of the valve clamping device 100 and facilitate the delivery. After the clamping arm 40 cooperates with the gripping arm 64 to clamp the leaflet, the leaflet is clamped in the receiving groove 45 to increase the contact area between the clamping arm 40 and the leaflet. The leaflet is pressed in the receiving groove 45 of the clamping arm 40, which can also increase the clamping force to the leaflet 300. One end of the connecting frame 42 adjacent to the fixing base 20 extends obliquely toward the proximal end and is hinged on the fixing base 20, that is, the connecting frame 42 has a hinge hole for a hinge inserted there through to be connected to the fixing base 20.

The clamping arm 40 is actuated by the actuator assembly 70 to open and close relative to the fixing base 20, and the included angle between the two clamping arms 40 can reach a maximum of 300 degrees, that is, after the clamping arm 40 is opened relative to the fixing base 20, it can be turned downward to a certain extent, which is facilitated to clamp the leaflet in motion and improves the success rate of clamping. If the clamping effect is found to be unsatisfactory after clamping, the leaflet can be loosened by turning the clamping arm 40 downward, and clamping again. In this embodiment, the included angle between the two clamping arms 40 is preferably 0-240 degrees, more preferably 120-180 degrees.

Preferably, an anti-slip structure (not shown in the figure) can be provided on the inner surface of the receiving groove 45 of the clamping arm 40 to enhance the frictional force when the clamping arm 40 is in contact with the leaflets, thereby providing a stable clamping force and avoiding the clamping arms 40 cause damage to the leaflets. The anti-slip structure may be a protrusion or groove provided on the inner surface of the receiving groove 45 of the clamping frame 44 or a gasket made of a biocompatible material with a high friction coefficient attached to the inner surface of the receiving groove 45.

Preferably, an active drug may be applied on the inner surface of the receiving groove 45 of the clamping arm 40 to promote the endothelial cells to crawl and grow on the inner surface of the clamping arm 40 and the gripping arm 64.

The actuator assembly 70 further includes a connection base 74 disposed at the distal end of the actuator shaft 72 and a pair of connecting rods 76 movably connected to both sides of the connection base 74. The actuator shaft 72 movably passes through the fixing base 20 and is connected to the connection base 74. One end of each connecting rod 76 is connected to a corresponding one of the clamping arms 40, and the other end is connected to the connection base 74 by pivoting, that is, each clamping arm 40 is connected to the connection base 74 of the actuator assembly 70 through the connecting rod 76 on the corresponding side.

In this embodiment, one end of the connecting frame 42 of each clamping arm 40 away from the clamping frame 44 is rotatably connected to the same position of the fixing base 20, and the connecting frame 42 of each clamping arm 40 is rotatably connected to the proximal end of the connecting rod 76 at the corresponding side, and the distal end of the connecting rod 76 is rotatably connected to the connection base 74 at the distal end of the actuator shaft 72 by means of pins or bolts. When the actuator shaft 72 slides toward the distal end relative to the fixing base 20 in the axial direction, the connecting rod 76 is driven to move, under the pulling force of the connecting rod 76, the clamping arm 40 rotates around the pin hole to open relative to the fixing base 20. When the actuator shaft 72 slides proximally relative to the fixing base 20 in the axial direction, the connecting rod 76 pulls the clamping arm 40 to rotate around the pin hole to close relative to the fixing base 20.

The connection base 74 includes two opposite first planes and two connecting surfaces connecting the two first planes, and two opposite ends of the connection base 74 are respectively provided with a pair of pin holes penetrating the two first planes. The pin holes are used to connect the clamping arms 40 by pin hinges. The cross-sectional dimension of the connection base 74 parallel to the second plane direction gradually decreases from the proximal end to the distal end, that is, the shape of the connection base74 is any structure such as a hemisphere, a spherical cap or a bullet shape, so that the valve clamping device 100 is easier to be pushed in the patient's body. The connection base 74 and the actuator shaft 72 may be of an integral structure or a non-integrated structure. In this embodiment, the connection base 74 and the actuator shaft 72 are non-integrated structures, the actuator shaft 72 is a round rod body, the distal end of the round rod body is provided with an external thread, and the actuator shaft 72 and the connection base 74 are screwed and then fixed by welding. In other embodiments, the actuator shaft 72 may be fixedly connected to the connection base 74 by other detachable or non-detachable connection methods such as snap-fit.

The outer peripheral surface of the actuator shaft 72 is provided with a positioning portion 720. The positioning portion 720 can protrusions protruding from a periphery of the actuator shaft 72, or grooves formed on the outer peripheral surface of the actuator shaft 72. In this embodiment, the positioning portion 720 includes a plurality of grooves 722 facing the locking hole 820 of the locking member 82; the edges of the locking hole 820 are engaged in the corresponding grooves 722, and the two play a role similar to mechanical engagement, which can avoid the edge of the locking hole 820 of the locking member 82 being subjected to force and therefore having wears, so as to ensure the stability of the locking. The shape of the groove 722 may be semicircular, rectangular, trapezoidal or triangular. The groove 722 in this embodiment is a semi-circular groove structure. The width of the groove 722 should be set in the range of 0.04-0.30 mm, preferably 0.08-0.20 mm. If the width of the groove 722 is too small, the embedding and overlapping amount of the two cannot be guaranteed, and there is a possibility of wear or slip. If the width of the groove 722 is too wide, the level of closing of the valve clamping device will be affected. Specifically, each groove 722 is provided with a circle along the circumferential direction of the actuator shaft 72, and a plurality of grooves 722 are arranged at the distal end of the actuator shaft 72 in the axial direction. Preferably, the plurality of grooves 722 are arranged in parallel, that is, evenly spaced along the axial direction of the actuator shaft 72.

In other embodiments, the positioning portion 720 includes a plurality of protrusions disposed facing the locking hole 820 of the locking member 82, and the edges of the locking hole 820 are snapped to the corresponding protrusions. Specifically, each protrusion is a flange provided with a circle along the circumferential direction of the actuator shaft 72, and several flanges are arranged at the distal end of the actuator shaft 72 in the axial direction. Preferably, several protrusions are arranged in parallel, that is, are evenly spaced along the axial direction of the actuator shaft 72. The width of the protrusion is in a range of 0.04-0.30 mm, preferably 0.08-0.20 mm.

As shown in FIG.5 to FIG.9 , the fixing base 20 includes a rectangular fixing frame 21, a connecting block 22 disposed at the proximal end of the fixing frame 21, fixing blocks 23 disposed on opposite sides of the fixing frame 21, and a protrusion 25 disposed in the inner cavity of the fixing base 20. The fixing base 20 is provided with a through hole 24 extending through the connecting block 22 and the fixing frame 21 in the axial direction, and the through hole 24 is used for inserting the actuator shaft 72 there through. The opposite ends of the connecting block 22 are respectively provided with pin holes 26. The axis of the pin holes 26 is perpendicular to the axis of the through hole 24. The connecting block 22 and the connecting frames 42 of the clamping arm 40 are connected to each other through the pin hole 26. The protrusion 25 is disposed on one of the side walls of the inner cavity of the fixing frame 21 , the proximal end of the protrusion 25 is provided with an inclined surface 251, the first end 823 of the locking member 82 abuts against the protrusion 25, and there is a gap 826 between the second end 825 of the locking member 82and the inner cavity of the fixing base 20 to facilitate the rotation of the second end 825 of the locking member 82 along the first end 823, and to prevent the locking member 82 from interfering with the fixing base 20 when unlocking, thereby affecting the unlocking effect. In the prior art, the end of the locking member 82 in contact with the protrusion 25 of the fixing base 20 is engaged in the inner wall of the fixing base 20 and connected with the inner wall of the fixing base 20, this structure will cause the pulling force increases when the locking member 82 is unlocked by pulling. In present application, the locking member 82 overlaps the surface of the protrusion 25 of the fixing base 20, thereby reducing the pulling force required for unlocking. A first rounded corner 253 is formed between the proximal end of the inclined surface 251 and the inner surface of the fixing frame 21, and a second rounded corner 255 is formed between the distal end of the inclined surface 251 and the side surface of the protrusion 25. Specifically, the first end 823 of the locking member 82 overlaps the first rounded corner 253, when the first end 823 of the locking member 82 is in contact with the inclined surface 251, the pushing member 84 abuts against the proximal end surface of the locking member 82, so that the distal end surface of the first end 823 is attached to the inclined surface 251, the locking member 82 is inclined relative to the actuator shaft 72, and the locking hole 820 of the locking member 82 is engaged with the positioning portion 720 of the actuator shaft 72, so that the actuator shaft 72 is relatively fixed with the fixing base 20.

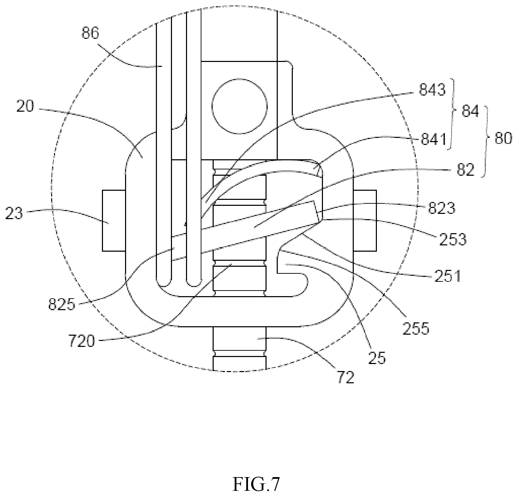

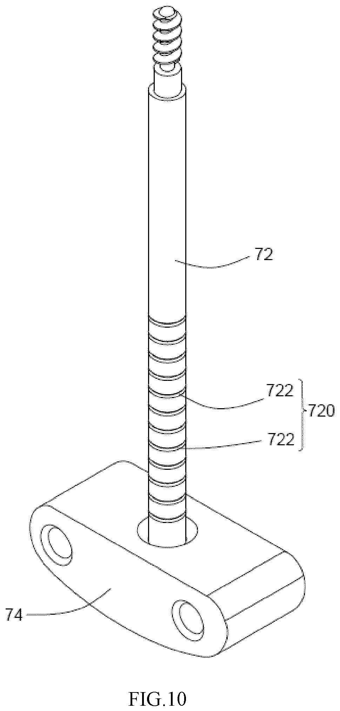

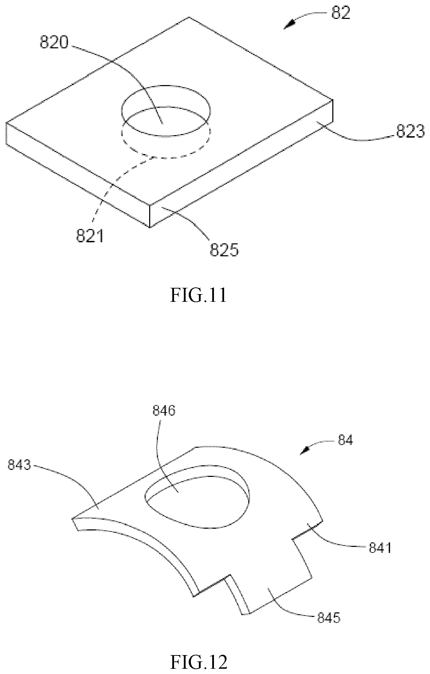

As shown in FIGS. 7-11, in this embodiment, the locking member 82 is a plate-like structure, and the locking member 82 includes a first end 823 and a second end 825 opposite to each other, the first end 823 abuts against the inside of the fixing base 20, the second end 825 can be rotated around the first end 823 until the axis of the locking hole 820 is coaxial with the axis of the fixing base 20, so as to facilitate the axial movement of the actuator shaft 72. When the locking member 82 is obliquely relative to the actuator shaft 72, so that the edge of the locking hole 820 is engaged to the positioning portion 720 of the actuator shaft 72, the axis of the locking hole 820 is inclined to the axis axial direction of the fixing base 20, and the pressing member 84 abuts against the proximal end surface of the locking member 82, the locking member 82 restricts the actuator shaft 72 to move in the axial direction. When the second end 825 of the locking member 82 rotates around the first end 823 to a position where the axis of the locking hole 820 and the axis of the fixing base 20 are coaxial or parallel, the locking member 82 releases the locking of the actuator shaft 72, and at this time, the actuator shaft 72 can move in the locking hole 820 in the axial direction.

As shown in FIG.7, FIG.9 and FIG.12, the pushing member 84 includes a first side 841 and a second side 843 opposite to each other, the first side 841 abuts against the inner wall of the fixing base 20, and the second side 843 abuts against the proximal end face of locking member 82. The pushing member 84 elastically pushes against the locking member 82 so that the locking member 82 is inclined relative to the actuator shaft 72, so as to ensure that the edge of the locking hole 820 is stuck to the positioning portion 720 of the actuator shaft 72. Specifically, the pushing member 84 is a sheet-like structure made of elastic material, the pushing member 84 further includes a middle portion connected between the first side 841 and the second side 843, and the middle portion of the pushing member84 is bent toward the proximal end and gradually abut against the inner wall of the fixing base 20 so that the pushing member 84 has a stable elastic force. In the initial state, the pushing member 84 is in the bent and deformed state after being pressed, thereby restricting the movement of the locking member 82 toward the proximal end. Preferably, correspondingly, the first side 841 of the pushing member 84 is provided with a latching piece 845, and the proximal end of the inner wall of the fixing frame 21 is provided with a latching slot 27 communicating with the inner cavity thereof for fixing the pushing member 84. The latching slot 27 is closer to the proximal end relative to the protrusion 25, so that the pushing member 84 can bend and abut the locking member 82. The latching piece 845 of the pushing member 84 are engaged in the latching slot 27 of the fixing base 20, so that the pushing member 84 is fixedly connected to the fixing base 20 to prevent the pushing member 84 from being displaced. A through hole 846 is defined in the middle of the pushing member 84 along the axial direction, and the actuator shaft 72 is inserted through the through hole 846 in the axial direction. In the prior art, the pushing member is recessed toward the distal end and then abuts against the proximal end surface of the locking member 82, and the opposite sides of the pushing member are engaged in the fixing base 20, when unlocking, to pull the locking member 82 needs the pushing member to be squeezed to deform, and at this time, a greater unlocking force is required to overcome the elastic force of the pushing member. In the present application, since the pushing member 84 is protruded from the proximal end of the locking plate toward the proximal end, and only one side abuts the proximal end surface of the locking member 82, the resistance to be overcome by pressing the pushing member is relatively small, that is, the pulling force required to unlock is less.

In this embodiment, the width of the protrusion 25 is smaller than the width of the base of the pushing member 84, and the width of the latching slot 27 of the fixing base 20 is also smaller than the width of the base of the pushing member 84. The latching piece 845 of the pushing member 84 is just inserted into the latching slot 27 of the fixing base 20, so that the locking member 82 will not be displaced under the unlocking force. Similarly, the locking member 82 will not be displaced during the process of returning to the locked state, so that both the unlocking effect and the locking effect can be guaranteed.

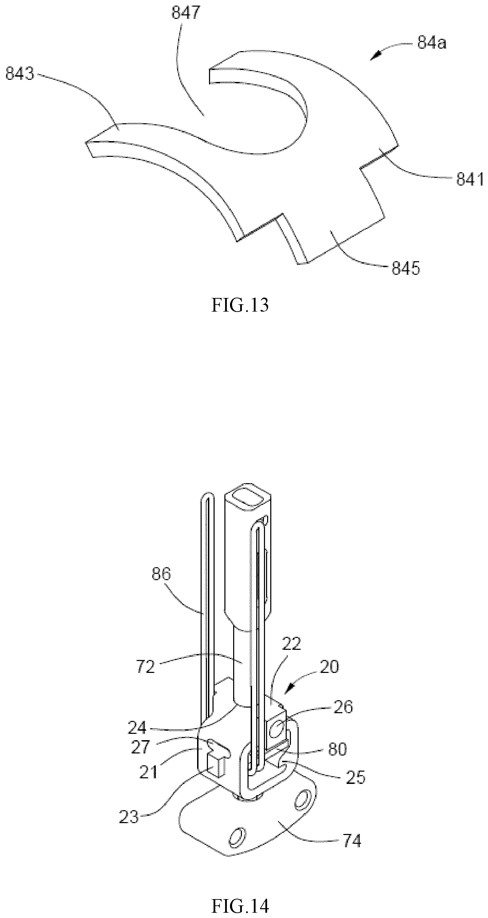

As shown in FIG. 13, in other embodiments, a notch 847 is formed in the middle of the pushing member 84a, the actuator shaft 72 is inserted through the notch 847 in the axial direction, and the end of the notch 847 facing away from the latching piece 845 penetrates the second side 843 of the pushing member 84a.

In other embodiments, the first side 841 of the pushing member 84 can also be directly clamped, welded or glued to the inner wall of the fixing base 20.

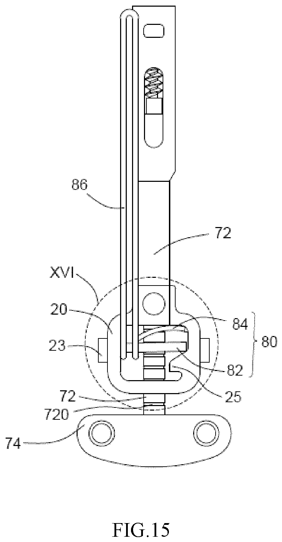

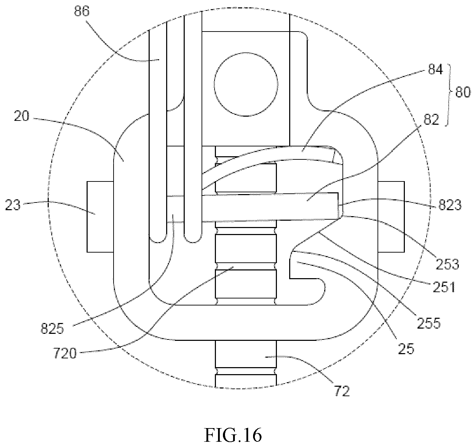

Please refer to FIGS. 5-6 and 14-18 together, the locking mechanism 80 further includes an unlocking member 86, the unlocking member 86 in this embodiment is one-side controlled, which means the unlocking member 86 attached to one side (second end 825) of locking member 82. Specifically, the unlocking member 86 is connected to the second end 825 of the locking member 82, when pulling the unlocking member 86 toward the proximal end causes the second end 825 of the locking member 82 to rotate around the first end 823 toward the proximal end, the pushing member 84 is elastically deformed. The deformation causes a gap between the outer peripheral surface of the actuator shaft 72 and the locking hole 820 and as well as the through hole 846 of the pushing member 84, so that the actuator shaft 72 can move in the axial direction. When the pulling force on the unlocking member 86 is released, the pushing member 84 returns to the initial state and the second end 825 of the locking member 82 rotates around the first end 823 toward the distal end, until the locking member 82 is locked on the positioning portion 720 of the actuator shaft 72, so that the actuator shaft 72 is relatively fixed to the fixing base 20. In the prior art, the unlocking member is a symmetrical open structure on both sides, in which only one side abuts the locking member, when the unlocking member is pulled, the side abutting the locking member moves toward the proximal end and pulls the locking member, and the other side is fixed, which is equivalent to losing a part of the pulling force, so the required unlocking force is greater. In this embodiment, the unlocking member 86 is arranged on one side of the actuator shaft 72, and the shape of the unlocking member 86 is a regular and symmetrical structure, when the force is applied, the two lines are simultaneously stressed, which can better ensure the transmission stability of the unlocking force, in this way, a smaller diameter unlocking member 86 can satisfy the unlocking force requirement.

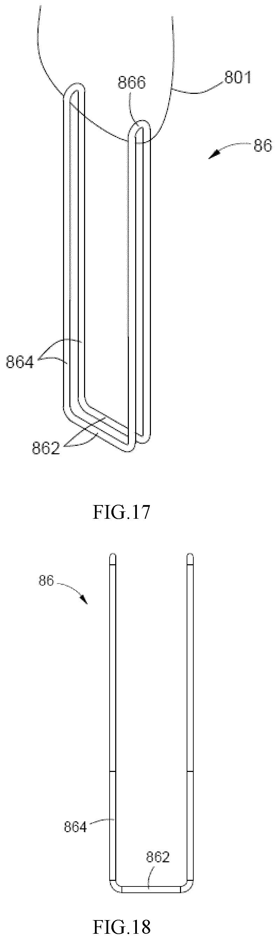

As shown in FIGS. 17 and 18, in this embodiment, the unlocking member 86 includes a double-line structure looped on the second end 825 of the locking member 82. Specifically, the double-line structures are arranged side by side and are both looped on the second end 825 of the locking member 82. Each row of double-line structures includes a connecting section 862 connected to the second end 825 of the locking member 82, and an extending section 864 extending from opposite ends of the connecting section 862 to the proximal end respectively, and the connecting section 862 and the extending section 864 enclose a U-shaped structure provided at the second end 825; the proximal end of each extending section 864 is connected to the proximal end of the adjacent extending section 864 through an arc-shaped joint segment 866. The unlocking member 86 in this embodiment is made of nickel-titanium line through heat-setting and crimped by stainless steel sleeve.

In order to facilitate remote control of the unlocking member 86 outside the patient's body, the proximal end of the unlocking member 86 is detachably connected to the control line 801. The distal end of the control line 801 extends out of the patient's body through the tube assembly of the delivery device 500. The control line 801 is usually made of polymer material. In this embodiment, the control line 801 is U-shaped, and penetrates the double-line structure of the unlocking member 86, that is, a gap is provided between the two adjacent extending sections 864 of the double-line structure adjacent to the joint section 866, and the control line 801 passes through two gaps in turn.

The tissue gripper 60 is made of at least in part a shape memory material and has been heat setting treatment. During production, the shape memory material is first cut into the desired shape by laser cutting, and then placed in a mold for heat setting treatment at about 550°C to make it have a specific shape. In a natural state, the gripping arms 64 on both sides of the tissue gripper 60 radially extend outward relative to the connecting frame 62.

Preferably, the included angle between the two clamping arms 64 in the natural unfolded state should be slightly greater than the included angle between the two clamping arms 40, that is, the included angle between the length direction of the clamping arms 64 and the axial direction of the fixing base 20 is greater than or equal to the included angle between the clamping arm 40 and the fixing base 20 when the clamping arm 40 corresponding to that side is fully opened with respect to the fixing base 20, so that the free end of each gripping arm 64 and the corresponding clamping arm 40 are close to each other and have a certain clamping force between each other, so as to provide a more stable clamping force. Specifically, in this embodiment, the included angle between the length direction of the clamping arms 64 and the axial direction of the fixing base 20 ranges from 0 to 150 degrees, that is, in a natural state, the angle between the two gripping arms 64 can be up to 300 degrees at most, preferably 160-200 degrees.

In this embodiment, the entire tissue gripper 60 is made of super-elastic nickel-titanium alloy, thereby reducing the difficulty of the production process, simplifying the process flow, and reducing the production cost. In addition, in other embodiments, the connecting frame 62 and the gripping arm 64 can be made separately and then fixedly connected, as long as the connection between the two has elasticity or shape memory performance, and can be retracted and rebounded relative to the fixing base 20.

In the present embodiment, two opposite side walls of the connecting frame 62 are respectively provided with connecting slot 622 (as shown in FIG. 19) for engaging with the fixing blocks 23 of the fixing base 20. The shape of the connecting slot 622 can be a rectangle, an ellipse, a prism or other shapes. In this embodiment, a rectangle is preferred for higher stability. The fixing base 20 is accommodated in the inner cavity of the connecting frame 62, and the actuator shaft 72 is inserted through the fixing base 20 and the connecting frame 62 through the opening of the proximal end of the connecting frame 62. After the connecting frame 62 cooperates with the fixing base 20, it can prevent the fixing base 20 from moving left and right, that is, it plays the role of left and right limit; and the fixing blocks 23 cooperates with the connecting slot 622, it can prevent the tissue gripper 60 and the fixing base 20 from moving back and forth, that is, it acts as a front and rear limit.

As shown in FIGS. 5-8 and 19-20, each gripping arm 64 is provided with at least one row of barbs 642 along its length. In this embodiment, two opposite sides of each gripping arm 64 are respectively provided with one row of barbs 642, and the number of one row of barbs 642 on each side is four. Preferably, the end of each barb 642 is rounded to avoid piercing the leaflets.

There is an angle A between each barb 642 and the gripping arm 64, and the angle A ranges from 30 to 85 degrees, preferably 45 to 65 degrees. Too large or too small angle A will increase the difficulty of capturing the valve leaflets. The angle of the included angle A between each barb 642 and the gripping arm 64 may be the same or different. In this embodiment, the included angle between each barb 642 and the gripping arm 64 is 30 degrees.

The effective length L of each barb 642 is in the range of 0.3-2.0 mm, preferably 0.5-1.2 mm. The effective lengths L of the barbs 642 may or may not be the same. In this embodiment, the extension length L of the barbs in each row of barbs 642 is the same, that is, the effective length L of each barb 642 is 0.8 mm.

In other embodiments, the included angle between the barbs in at least one row of barbs 642 of each gripping arm 64 and the corresponding gripping arm 64 gradually increases along the extending direction of the gripping arm 64. The effective length of the barbs in the at least one row of barbs 642 of the gripping arm 64 gradually increases from the proximal end to the distal end. The reason for this setting is that the thickness of the leaflets is not uniform, the edge of the leaflet is the thinnest, and the thickness gradually increases to the position where the leaflet connects with the annulus. Therefore, in accordance with the increased thickness from the edge of the leaflet to a middle position of the leaflet, and in order to ensure the force depth of each barb 642 at different contact positions with the leaflet tissue is approximately the same, and ensure the clamping force of the gripping arm 64 on the leaflet and does not pierce the leaflet, the angle and length of the barbs 642 are adjusted to adapt to the degree of force of the leaflet tissue of different thicknesses.

As shown in FIGS. 19 and 20, the free end of each gripping arm 64 is provided with a hole 644 for connecting the operating line 501 of the delivery device 500, and the free end of the gripping arm 64can be controlled by the operating line 501 extending outside the patient's body. In the delivery state, the free end of the gripping arm 64 is pulled by the operating line 501 and adheres to the surface of the fixing base 20; and after the control of the free end by the operating line 501 is released, the gripping arm 64 is released, and the gripping arm 64 returns to its natural state due to its own elastic memory performance, and presses the leaflet against the clamping arm 40. The operating line 501 may be a metal line made of nickel-titanium alloy or the like, and since it has nothing to do with the improvement and creation of the present application, it will not be repeated here.

In order to ensure the safety after implantation, the fixing base 20 and the clamping arm 40 are respectively made of biocompatible metal materials such as stainless steel, cobalt alloy, cobalt-chromium alloy, titanium alloy or nickel-titanium alloy. The actuator assembly 70 is made of polyester, silicone resin, stainless steel, cobalt alloy, cobalt chromium alloy or titanium alloy and other biocompatible polymer materials or metal materials. In this embodiment, the fixing base 20, the clamping arm 40 and the actuator assembly 70 are all made of stainless steel. The locking member 82 and the pushing member 84 are also made of biocompatible materials, the locking member 82 is preferably made of stainless steel or cobalt-chromium alloy with higher hardness, and the pushing member 84 is made of an elastic nickel-titanium alloy.

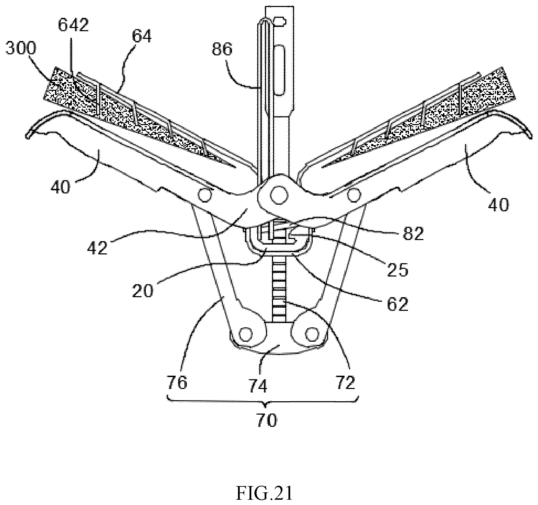

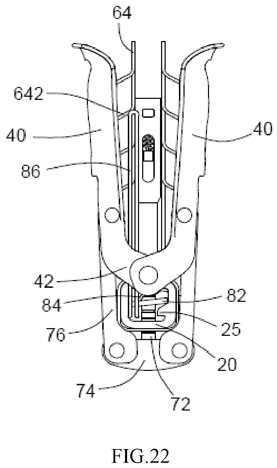

The following takes the mitral valve repair process as an example to describe the operation method of the valve clamping device with a locking mechanism of the present application, which mainly includes the following steps:
Step 1: the valve clamping device 100 is detachably connected to the distal end of the delivery device 500, and the operating line 501 connected to the gripping arm 64 is pulled toward the proximal end to control the gripping arm 64 to retract relative to the fixing base 20, so that the gripping arm 64 is fitted on the surface of the fixing base 20; and the control line 801 connected to the unlocking member 86 is pulled toward the proximal end, so that the locking member 82 unlocks the actuator shaft 72. Then, the actuator shaft 72 is moved toward the proximal end to actuate the connecting rod 76 to drive the clamping arm 40 to close relative to the fixing base 20, so that the valve clamping device 100 is in a fully retracted state, and then the pulling force on the control line 801 is released, and the pushing member 84 pushes against the second end 825 of the locking member 82 to rotate around the first end 823 toward the distal end until the locking member 82 is locked on the positioning portion 720 of the actuator shaft 72 , to keep the retracted state of the clamping arm 40 unchanged.
Step 2: after femoral vein puncture and transseptal puncture, advancing the distal end of the delivery device and the valve clamping device 100 from the left atrium to pass through the mitral valve into the left ventricle using the adjustable sheath.
Step 3: adjust the relative positions of the valve clamping device 100 and the mitral valve, so that the valve clamping device 100 is close to the anterior and posterior leaflets of the mitral valve.
Step 4: pull the control line 801 toward the proximal end to release the locking of the actuator shaft 72 by the locking member 82, and then move the actuator shaft 72 toward the distal end, thereby actuating the connecting rod 76 to drive the clamping arm 40 to open relative to the fixing base 20, then the pulling force on the control line 801 is released, and the second end 825 of the pushing member 84 pushes against the second end 825 of the locking member 82 to rotate around the first end 823 toward the distal end until the locking member 82 is locked on the positioning portion 720 of the actuator shaft 72.
Step 5: withdraw the valve clamping device 100 toward the proximal end, so that the clamping arm 40 holds the leaflet in the left ventricular side.
Step 6: release the control of each operating line 501 to the corresponding gripping arm 64 to release the gripping arms 64 on both sides, and the gripping arms 64 on each side press the leaflet 300 in the atrial side and cooperate with the clamping arm 40 to hold the leaflets (as shown in FIG.21).
Step 7: pull the control line 801 connected to the unlocking member 86 towards the proximal end so that the second end 825 of the locking member 82 rotates around the first end 823 toward the proximal end until the locking member 82 releases the locking of the actuator shaft 72. Then move the actuator shaft 72 toward the proximal end, and the actuator shaft 72 actuates the connecting rod to drive the clamping arm 40 to close relative to the fixing base 20 until the valve clamping device 100 is fully retracted. Release the pulling force on the control line 801 until the locking member 82 is locked in the positioning portion 720 of the actuator shaft 72 to make the actuator shaft 72 relatively fixed to the fixing base 20 (as shown in FIG.22).
Step 9: release the connection between the valve clamping device 100 and the delivery device, the control line 801, and the operating line 501, and withdraw the delivery device and the control line 801, and the operating line 501 from the patient's body. At this time, the anterior and posterior leaflets of the mitral valve are pulled toward each other by the valve clamping device 100 to obtain a double-orifice mitral valve, and the edge-to-edge repair of the mitral valve is completed, and the valve clamping device 100 is indwelled in the patient.

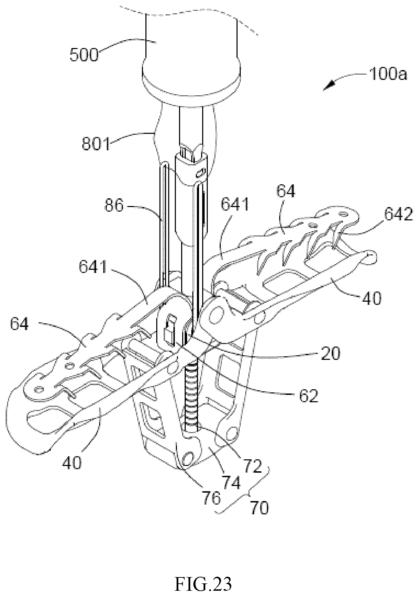

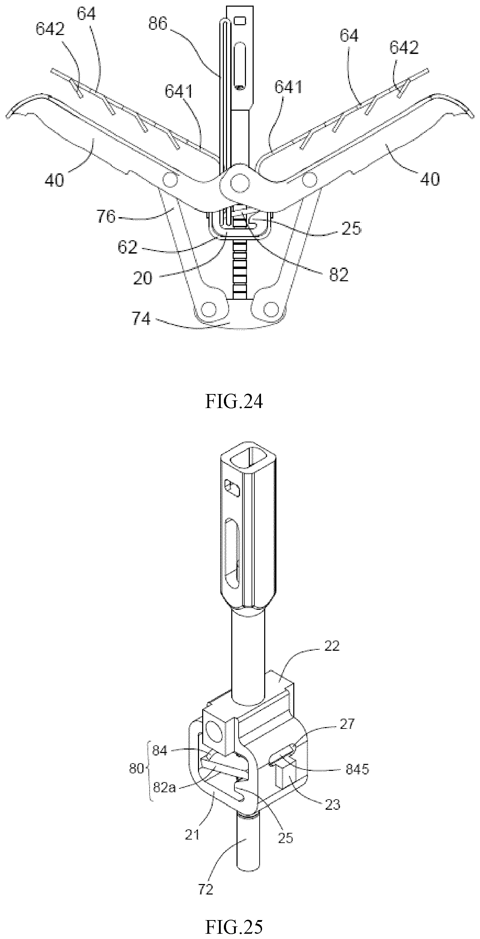

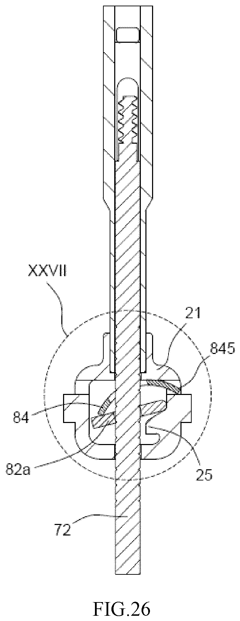

Please refer to FIG.23 to FIG.27 together. The structure of the valve clamping device 100a provided in the second embodiment of the present application is similar to the valve clamping device 100 provided in the first embodiment, while the difference is that in the second embodiment, the side wall of the first end 823a of the locking member 82ais an arc surface 826, which can improve the force between the locking member 82 and the contact point of the fixing base 20, and prevent the metal chips from falling off and the fatigue of the contact point due to the right-angle force. In addition, each clamping arm 64 is connected with the connecting frame 62 by a bending segment 641, and the width of the bending segment 641 is smaller than that of the connecting frame 62, and is smaller than the width of the clamping arm 64.

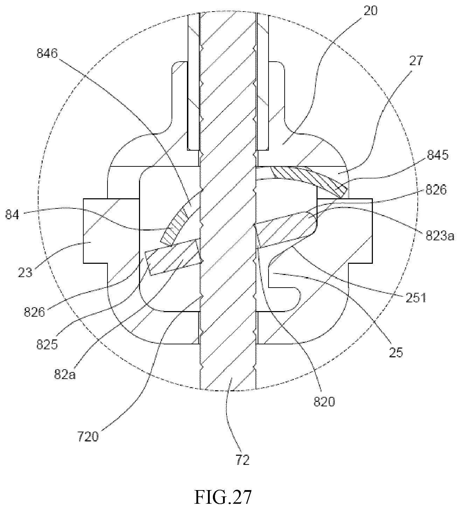

Specifically, as shown in FIG.27, when the first end 823a of the locking member 82 abuts against the protrusion 25, the arc surface 826 contacts the inclined surface 251 and the inner wall of the fixing base 20 at the same time, thereby facilitating the second end 825 of the locking member 82 rotates around the first end 823a. In this embodiment, the arc surface 826 of the locking member 82 is in contact with the protrusion 25, which can reduce the frictional force when the locking member 82 and the fixing base 20 rotate relative to each other, that is, reduce the unlocking force of the unlocking member 86, and also reduce the elastic force of the pushing member 84 to restore the locking state, thereby improving the locking and unlocking performance. Furthermore, since the arc surface 826 contacts the inclined surface 251 of the protrusion 25 and the inner wall of the fixing base 20 at the same time, there are two contact lines between the arc surface 826 of the locking member 82 and the inner wall of the fixing base 20, that is the arc surface 826 and the inclined surface 251 contacts the flat surface of the inner wall of the fixing base 20 at simultaneously, and the contact lines on the flat surface can ensure that the locking member 82 will be no displacement during the unlock process, thus ensuring the unlocking stability.

As shown in FIG.23, the width of the bending section 641 in this embodiment is smaller than the width of the connecting frame 62 and smaller than the width of the clamping arm 64 , which can not only reduce the weight of the valve clamping device 100a, but also facilitate rebound of the clamping arm 64, reduce the difficulty of clamping, improve the fatigue resistance of the valve clamping device implanted in the human body for a long time, and can also reduce the pulling force of the operating line 501 to pull the gripping arm 64 to fit the central axis, thereby reducing the reverse force applied on the operating line 501, preventing the operating line 501 from breaking, and improving the safety and effectiveness of the device.

Preferably, the bending section 641 is a reduced diameter structure. Specifically, the bending section 641 includes a first end connected to the connecting frame 62 and a second end connected to the clamping arm 64, and a width of the first end is greater than the width of the second end. The width of the bending section gradually decreases from the first end to the second end.

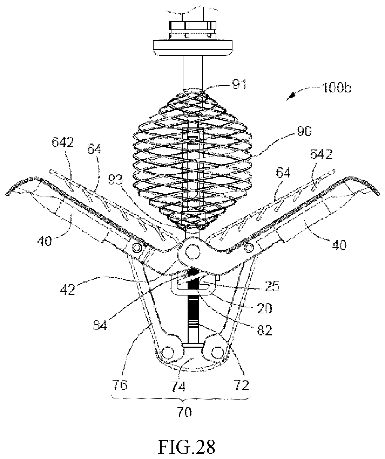

Referring to FIG.28, the structure of the valve clamping device 100b provided in the third embodiment of the present application is similar to the valve clamping device 100 provided in the first embodiment, the difference is: the valve clamping device 100b in the third embodiment also includes an adjusting member 90, which is arranged outside the fixing base 20. When the valve clamping device 100b clamps the valve, the adjusting member 90 is elastically clamped between a pair of gripping arms 64, and the adjusting member 90 is used to adjust the level of pulling between the two leaflets when the pair of clamping arms 40 is closed.

In this embodiment, the adjusting member 90 is made of elastic material, the distal end of the adjusting member 90 is fixedly connected to the fixing base 20, and the proximal end of the adjusting member 90 is suspended. Specifically, the adjusting member 90 includes a first end 91 and a second end 93 opposite to the first end 91, the first end 91 is the proximal end of the adjusting member 90, and the second end 93 is the distal end of the adjusting member 90. Wherein, the first end 91 is open, and the second end 93 is closed by a head. The head of the second end 93 are fixed to the fixing base 20 by common detachable or non-detachable connection methods such as welding, bonding, screw connection, crimping, bolt locking, etc. The welding connection is adopted in this embodiment.

The adjusting member 90 includes an elastic body. When the valve clamping device 100b is closed, the elastic body is filled between the anterior leaflet and the posterior leaflet of the mitral valve and abuts against the clamping arm 40, so it has the following advantages: (1) the elastic body has a buffering effect on the pulsating leaflets, so that the degree of pulling of the leaflets by the valve clamping device 100b can be adjusted to avoid leaflets damage; (2) the elastic body can follow the pulsation of the leaflets and be squeezed compression deformation, the generated elastic force pushes the part of the leaflet close to the elastic body to move away from the fixing base 20, so that the clamping angle between the anterior leaflet and the posterior leaflet of the mitral valve is smaller than the opening angle between the clamping arms 40 can reduce the pulling of the two leaflet by the valve clamping device 100b, so that the pulling degree of the two leaflet by the valve clamping device 100b is always kept within a reasonable range; (3) the elastic body can buffer the blood directly scouring to the internal of the valve clamping device 100b, thus preventing the valve clamping device 100b from falling off due to the continuous scouring of the blood, and also prevents blood from accumulating and forming thrombus in the dead corner between the clamping parts of the valve clamping device 100b; (4) when the elastic body is affected by the leaflets, a certain degree of deformation will occur, and the deformation degree increases with the increase of the pressure, so as to prevent the elastic body from being squeezed by the clamping arm 40 and acting on the clamping arm 40 after the leaflet is captured, this is ensured that the clamping effect of the valve clamping device 100b on the leaflets after release is consistent with that before release.

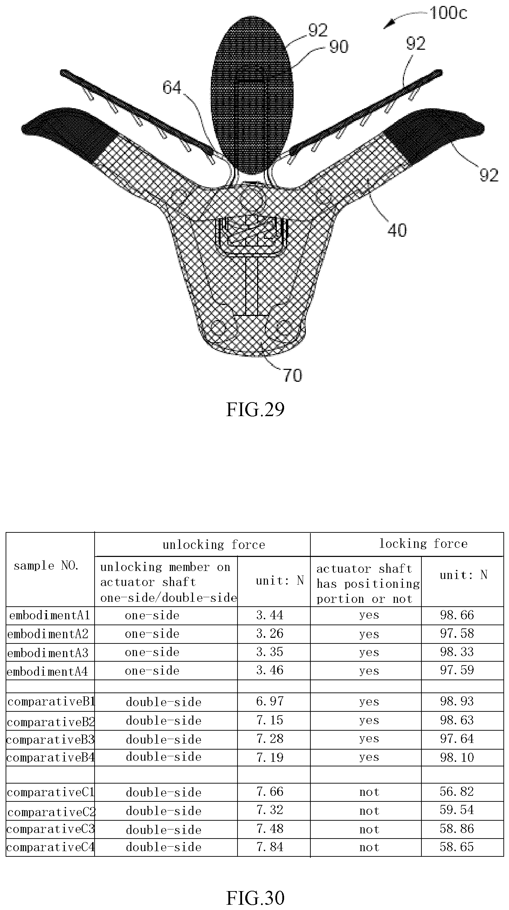

Referring to FIG.29, the structure of the valve clamping device 100c provided by the fourth embodiment of the present application is similar to the valve clamping device 100b provided by the third embodiment, and the difference lies in: the exterior and/or interior of the adjusting member 90, the gripping arm 64 and/or the valve clamping device 100b are provided a biocompatible mesh membrane 92. The mesh membrane 92 is a woven mesh structure with a plurality of holes. Wherein, the adjusting member 90 with the mesh membrane 92 can not only increase the biocompatibility, avoid tissue allergy and inflammatory reaction, and improve the product safety, but also can form an artificial barrier on the atrial side of the leaflet, block the thrombus in the blood, and close the opening of the entire valve clamping device 100c facing the side of the atrium, so as to avoid the repeated flushing of blood at the inner dead corner of the valve clamping device 100c to form thrombus.

The mesh membrane 92 can be made of polyethylene terephthalate, polypropylene, polytetrafluoroethylene, polyurethane and other polymer materials, and covers the outside of the adjusting member 90, the gripping arm 64 and the clamping arm 40. The inner mesh membrane material may be the same or different. In this embodiment, all three are made of PET, and all cover the outside of the adjusting member 90, the gripping arm 64 and the clamping arm 40.

The difference between the unlocking force and the locking force of the present application compared with the prior art is illustrated by the tensile test as follows, and the results are shown in FIG.30:
Three groups of valve clamping devices were produced respectively, wherein the first group (A1-A4) was the valve clamping device 100 according to the first embodiment of the present application, the actuator shaft 72 was provided with a positioning portion 720, and one side of the locking member 82 was overlapped on the inner wall of the fixing base 20, and a one-sided unlocking member is adopted. In the second group of comparative embodiments (B1-B4), the actuator shaft of the valve clamping device is also provided with a positioning portion, and one side of the locking member is overlapped with the inner wall of the fixing base, but the unlocking member adopts a double-sided structure. The third group of comparative embodiments (C1-C4) are the valve clamping devices of the prior art, with the outer peripheral surface of the actuator shaft has no positioning portion, and one side of the locking member is engaged in the inner wall of the fixing base, thereby securing the connection. The following performance tests were performed on the three groups of valve clamping devices:
1. Unlocking force test (unlocking force test of the control line)

The smooth performance of opening and closing of the valve clamping devices of embodiments A1 to A4, comparative embodiments B1 to B4, and comparative embodiments C1 to C4 were respectively tested.

Test equipment: HY-0580 electronic universal tensile testing machine produced by Shanghai Hengyi Precision Instrument Co., Ltd.

Test method: the valve clamping device is connected to the delivery device 500, the control line 801 is connected to the unlocking member 86 and pass through the proximal end of the delivery device 500, and the push shaft on the delivery device 500 is connected to the actuator shaft 72 of the valve clamping device, and the push shaft can be operated at the proximal end of a simple handle. The simple handle is fixed on the machine table of the pulling machine, the moving end of the pulling machine hooks the proximal end of the control line 801, and the pulling machine exerts an unlocking force on the control line 801, operate the push shaft at the proximal end of the simple handle, records the unlocking force required for the push shaft to smoothly open and close the valve clamping device.

### 2. Locking force test (self-locking force test of actuator shaft and locking member)

The valve clamping devices of embodiments A1-A4, comparative embodiments B1-B4, and comparative embodiments C1-C4 were tested for locking force (i.e., the locking force between the actuator shaft and the locking member), respectively.

Test method: the valve clamping device is connected to the simple handle, a test line is passed through the connecting base 74 of the actuator shaft 72, the simple handle is fixed on the machine table, the moving end of the tensile testing machine is hooked to the distal end of the test line. The moving end is then moved at a constant speed of 4.5 mm/min, and the force value is recorded when the actuator shaft 72 and the locking member 82 fail to slip.

It can be seen from the test result table in FIG.30 that the unlocking force required for the one-sided unlocking member in the first group of embodiments A1 to A4 is smaller and the locking force is stronger.

It should be noted that the above contents are all described by taking the valve clamp used for reducingor treating "mitral regurgitation" as an example. It can be understood that, in other embodiments, the valve clamp can also be used to reduceor treat "tricuspid regurgitation", and its principle and structure are the same as those used in the embodiments of the present application to solve "mitral regurgitation". The principle and structure of the valve clamp are basically the same. It is only necessary to form multiple clamps by multiple sets of proximal clamps and distal clamps, and each clamp can clamp a leaflet, which will not be repeated here.

Obviously, in other embodiments, the valve clamp provided by the present application can also be applied to other minimally invasive surgical operations that need to clamp more than three sheet-shaped valves together.

It should be noted that, on the premise of not departing from the principles of the embodiments of the present application, the specific technical solutions in the above embodiments are applicable to each other, which will not be repeated here.

The above are the implementations of the embodiments of the present application. It should be pointed out that for those of ordinary skill in the art, without departing from the principles of the embodiments of the present application, several improvements and modifications can also be made. It is regarded as the protection scope of this application.

## Claims

1. A valve clamping device with a locking mechanism, comprising:
a fixing base;
at least one pair of clamping arms, the at least one pair of clamping arms being connected to the fixing base and can be opened and closed relative to the fixing base;
an actuator assembly, the actuator assembly comprising a actuator shaft movably inserted in the fixing base, the actuator shaft moving in an axial direction to actuate the clamping arm to open and close relative to the fixing base, with a positioning portion being provided on the outer peripheral surface of the actuator shaft; and
a locking mechanism, the locking mechanism comprising a locking member and a pushing member, the locking member being provided with a locking hole in the axial direction, the actuator shaft being inserted into the locking hole, and the pushing member abutting against the locking member and being obliquely arranged in the fixing base so that the edge of the locking hole is engaged with the positioning portion.

2. The valve clamping device according to claim 1, wherein the positioning portion comprises a plurality of grooves and/or protrusions disposed facing the locking hole, and the edge of the locking hole is engaged into the groove and/or protrusion.

3. The valve clamping device according to claim 2, wherein the plurality of grooves or protrusions are arranged in parallel.

4. The valve clamping device according to claim 2, wherein a width of the groove or the protrusion is in a range of 0.04-0.30 mm.

5. The valve clamping device according to claim 1, wherein the locking member comprises a first end and an opposite second end, the first end abuts against the inner cavity of the fixing base, and the second end can rotate around the first end to a position where the axis of the locking hole is coaxial with the axial direction of the fixing base.

6. The valve clamping device according to claim5, wherein the inner cavity of the fixing base is provided with a protrusion, the first end of the locking member abuts against the protrusion, and there is a gap between the second end of the locking member and the inner cavity of the fixing base.

7. The valve clamping device according to claim 6, wherein the proximal end of the protrusion is provided with an inclined surface, the first end of the locking member overlaps the inclined surface, and the pushing member abuts against the proximal surface of the locking member so that the distal end surface of the first end is attached to the inclined surface.

8. The valve clamping device according to claim 7, wherein the side wall of the first end is an arc surface.

9. The valve clamping device according to claim 8, wherein the arc surface contacts the inclined surface and the inner wall of the fixing base simultaneously.

10. The valve clamping device according to claim 1, wherein the pushing member includes opposite first side and second side, the first side abuts against the inner wall of the fixing base, and the second side abuts against the proximal end surface of the locking member.

11. The valve clamping device according to claim 10, wherein the pushing member is made of elastic material, and the pushing member further comprises a middle part connected between the first side and the second side, the middle part bends toward the proximal end, and gradually abuts against the inner wall of the fixing base.

12. The valve clamping device according to claim 11, wherein a latching piece is provided on the first side of the pushing member, and a latching slot is provided on the inner wall of the fixing base, and the latching piece is engaged in the latching slot.

13. The valve clamping device according to claim 10, wherein a through hole is defined in the middle of the pushing member along the axial direction, and the actuator shaft is inserted through the through hole in the axial direction.

14. The valve clamping device according to claim 5, wherein the locking mechanism further comprises an unlocking member, the unlocking member is connected to the second end of the locking member, and the unlocking member is pulled toward the proximal end so that the second end of the locking member rotates around the first end.

15. The valve clamping device according to claim 14, wherein the unlocking member comprises a double-line structure, and the double-line structures are arranged side by side and are both looped on the second end of the locking member.

16. The valve clamping device according to claim 1, wherein the valve clamping device further comprises at least one pair of gripping arms, the gripping arms are arranged between the fixing base and the at least one pair of clamping arms, each of the gripping arms cooperates with the clamping arm at the corresponding side to grip the valve.

17. The valve clamping device according to claim 16, wherein the valve clamping device further comprises an adjusting member, and the adjusting member is arranged the outside of the fixing base.

18. The valve clamping device according to claim 17, wherein the adjusting member is made of elastic material, the distal end of the adjusting member is fixedly connected to the fixing base, and the proximal end of the adjusting member is suspended.

19. The valve clamping device according to claim 17, wherein a biocompatible mesh membrane covers the exterior and/or interior of the adjusting member, the gripping arm and/or the clamping arm.

20. A valve repair system, comprising the valve clamping device according to any one of claims 1-19, and a delivery device detachably connected to the valve clamping device, wherein the delivery device comprises an operating line, and the distal end of the operating lineand the unlocking member of the locking mechanism are detachably connected.
